# EUROPEAN PATENT APPLICATION

(11) **EP 1 760 075 A1**
(43) Date of publication of application: **07.03.2007**
(21) Application number: 05751221.2
(22) Date of filing: 13.06.2005
(51) Int. Cl.: C07D 207/14, C07C 231/02, C07C 231/12, C07C 235/52, C07D 403/06

(54) **METHOD FOR PRODUCING ACETAMIDOPYRROLIDINE DERIVATIVE AND INTERMEDIATE COMPOUND THEREOF**

(30) Priority: 14.06.2004 JP 2004175159; 14.06.2004 JP 2004175158
(71) Applicant: Teijin Pharma Limited, Tokyo 100-0011 (JP)
(72) Inventor: KAWANA, A. Teijin Pharma Ltd.,Iwakuni Research Ctr, Iwakuni-shi, Yamaguchi 7400014 (JP); TAKEYASU,T. Teijin Pharma Ltd,Iwakuni Research Ctr, Iwakuni-shi,Yamaguchi 7400014 (JP); HAZATO, A. Teijin Pharma Ltd,Iwakuni Research Ctr., Iwakuni-shi, Yamaguchi 7400014 (JP)
(74) Representative: Hallybone, Huw George
(86) International application number: PCT/JP2005/011187
(87) International publication number: WO 2005/121081

(57) **Abstract**

A production method of an acetamidopyrrolidine derivative or a salt thereof comprising the reaction steps represented by the following formula: 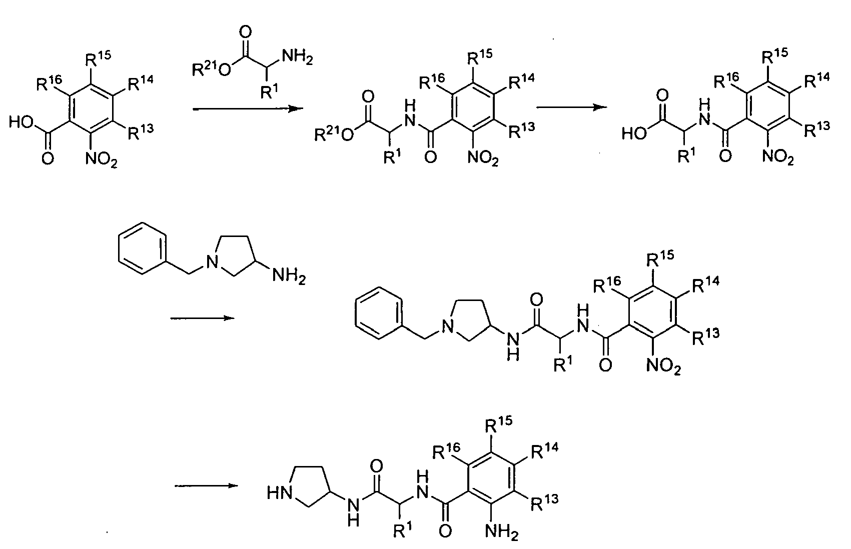 wherein R¹ represents a hydrogen atom or a C₁-C₆ alkyl group; R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group; and R²¹ represents an ester-type protecting group.

## Description

### Field of the Invention

The present invention relates to a method for producing acetamidopyrrolidine derivatives. More specifically, it relates to benzamidoacetic acid derivatives and acetamidopyrrolidine derivatives valuable for synthesizing chemokine receptor antagonists that are expected as effective for treating and/or preventing diseases in which infiltration of leukocyte components such as monocyte and lymphocyte into tissues plays a major role in their progress and preservation; and a production method thereof.

### Background Art

Chemokines such as MIP-1α and MCP-1 are protein factors that cause migration and activation of leukocytes. It is known that their function is expressed via mediation by chemokine receptors on leukocytes (Allergy & Immunology, 1999, vol. 6, no. 11). It is, therefore, expected that a chemokine receptor antagonist, which can inhibit activity of chemokines onto target cells, is effective for treating and/or preventing one or more diseases in which infiltration of leukocytes into tissues plays a major role in their progress and preservation, such as arteriosclerosis, rheumatoid arthritis, psoriasis, asthma, ulcerous colitis, nephritis (nephropathy), multiple sclerosis, pulmonary fibrosis, cardiomyopathy, hepatitis, pancreatitis, sarcoidosis, Crohn's disease, endometriosis, congestive cardiac failure, viral meningitis, cerebral infarction, neuropathy, Kawasaki disease, septicemia, allergic rhinitis, allergic dermatitis, and the like (Schwarz, M.K. et al., Exp. Opin. Ther. Patents, 1999, 9, 1471). On the basis of these findings, investigation towards development of chemokine receptor antagonists has progressed, and cyclic amine derivatives having high activity as chemokine receptor antagonists were found (WO 99/25686 pamphlet).

Because many of preferred compounds having chemokine receptor antagonist activity contain acetamidopyrrolidine skeletal structure and/or anthranilamide skeletal structure, in order to obtain a wide variety of compounds having chemokine receptor antagonist activity, it was desired to produce such synthetic intermediates efficiently by a method suitable for large-scale synthesis.

Examples of compound having such acetamidopyrrolidine skeletal structure are disclosed in WO 01/96303 pamphlet. From these compounds, however, the above-mentioned compounds having chemokine receptor antagonist activity cannot be derived.

As a production method for obtaining compounds having acetamidopyrrolidine skeletal structure, production examples for compounds with similar structure are described in the publication of WO 99/25686 or Japanese Patent No. 2001-500891. However, in the case of introducing a valuable substituent into a side chain of pyrrolidine, the production methods disclosed in these documents are not simply applied, and furthermore the disclosed reaction examples are not necessarily suitable for large-scale synthesis.

As examples of compounds with similar structure to the benzamidoacetic acid derivatives, which are production intermediates of the compounds having anthranilamide skeletal structure, Japanese Patent Application Laid-open No. H5-169812 discloses monosubstituted nitrobenzamide derivatives, Japanese Patent Application Laid-open No. 2002-357285 discloses acylated aminoacetonitrile derivates, and Japanese Patent Application Laid-open No. 2002-326980 discloses diamide derivatives. However, the above-mentioned compound having chemokine receptor antagonist activity cannot be derived from these compounds.

Although compounds having an amide structure are disclosed in WO 02/60859 pamphlet and WO 02/50019 pamphlet as compounds having chemokine receptor inhibitory activity, no compounds with anthranilic acid structure have been specifically disclosed. That is, it has not been suggested that the benzamidoacetic acids of the present invention could be used for producing compounds having chemokine receptor inhibitory activity.

### DISCLOSURE OF THE INVENTION

An object of the present invention is to find out production intermediates for the compounds disclosed in WO 99/25686 pamphlet. Particularly, the present invention has as an object to provide production intermediates suitable for constructing the anthranilamide skeletal moiety and production intermediates in upper stream in the production process. Furthermore, it has as another object to provide a method suitable for industrial production thereof.

In other words, objects of the present invention are to provide a production method of the above-mentioned compounds in which mild conditions can be employed, less environmental impact is imposed and there are economical advantages, such as no need of special equipments, easiness of operation, less number of steps, high yields and the like, and to provide intermediates to realize such a method.

The present investors have investigated with the above objects, found production methods of the present invention and a production intermediates therein, and attained the following inventions.

Namely, the first invention is a production method of an acetamidopyrrolidine derivative or a salt thereof comprising a reaction step represented by the following formula:

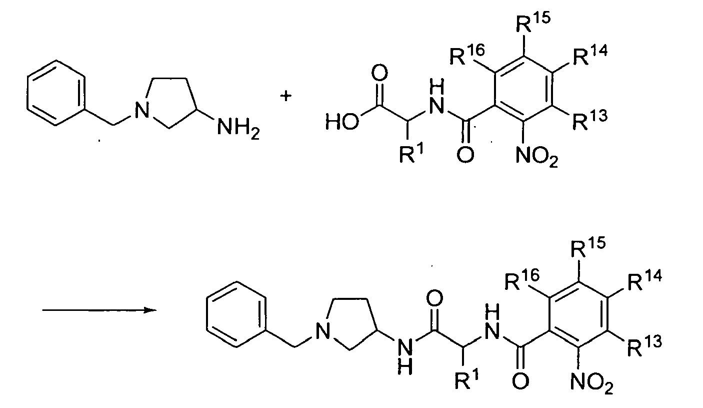

In the formula, R¹ represents a hydrogen atom or a C₁-C₆ alkyl group and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time. Here one or both of the starting materials, i.e., the aminopyrrolidine derivative and the benzamidoacetic acid derivative, may be a form of salt. Similarly, the product of the method of the present invention can form a salt. The present invention also includes a case in which the product is obtained as a salt and a case in which the product is transformed to a salt after the reaction.

Because the acetamidopyrrolidine derivative thus obtained has a chiral carbon in the pyrrolidine ring, enantiomers may exist. The present invention includes methods for producing any of R-form, S-form and mixtures thereof. Further, when R¹ represents a C₁-C₆ alkyl group, there may exist enantiomers with respect to the carbon atom bonding to R¹. The present invention also includes methods for producing any of the R-form, the S-form and mixtures thereof.

The second invention is a production method of an acetamidopyrrolidine derivative or a salt thereof comprising a reaction step represented by the following formula:

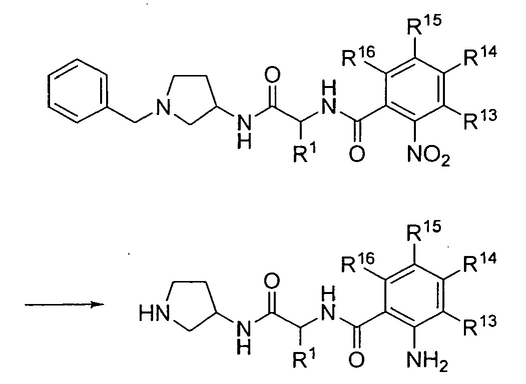

In the formula, R¹ represents a hydrogen atom or a C₁-C₆ alkyl group and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time.

Here the starting material, i.e., the acetamidopyrrolidine derivative, which has a basic group, may be a form of salt. Similarly, the product of the method of the present invention can form a salt. The present invention also includes a case in which the product is obtained as a salt and a case in which the product is transformed to a salt after the reaction.

Because the acetamidopyrrolidine derivative thus obtained has a chiral carbon in the pyrrolidine ring, enantiomers may exist. The present invention includes methods for producing any of R-form, S-form and mixtures thereof. Further, when R¹ represents a C₁-C₆ alkyl group, there may exist enantiomers with respect to the carbon atom bonding to R¹. The present invention also includes methods for producing any of the R-form, the S-form and mixtures thereof.

The third invention is a production method of a benzamidoacetic acid derivative comprising a reaction step represented by the following formula:

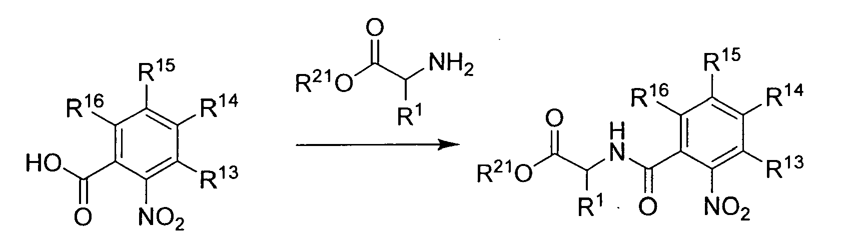

In the formula, R¹ represents a hydrogen atom or a C₁-C₆ alkyl group; R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group; and R²¹ represents an ester-type protecting group, excluding a case in which all of R¹³, R¹⁴, R¹⁵ and R¹⁶ are hydrogen atoms at the same time.

When R¹ represents a C₁-C₆ alkyl group, there may exist enantiomers with respect to the carbon atom bonding to R¹. The present invention includes methods for producing any of the R-form, the S-form and mixtures thereof.

Further, because the amino acid ester derivative and the nitrobenzoic acid derivative used in this reaction contain an amino group and a carboxyl group, respectively, both of them can form a salt. The present invention also includes a case in which one or both of these starting materials are in a form of salt.

The fourth invention is a production method of a benzamidoacetic acid or a salt thereof comprising a reaction step represented by the following formula. The product of this reaction is used as the starting material in the first invention.

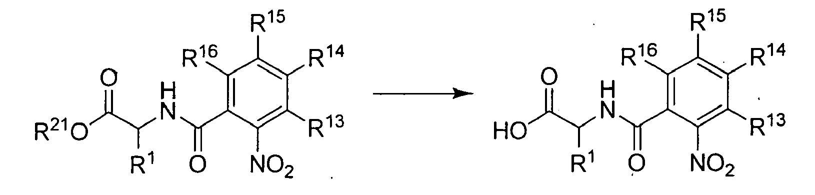

In the formula, R¹ represents a hydrogen atom or a C₁-C₆ alkyl group; R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group; and R²¹ represents an ester-type protecting group, excluding a case in which all of R¹³, R¹⁴, R¹⁵ and R¹⁶ are hydrogen atoms at the same time.

When R¹ represents a C₁-C₆ alkyl group, there may exist enantiomers with respect to the carbon atom bonding to R¹. The present invention includes methods for producing any of the R-form, the S-form and mixtures thereof.

The benzamidoacetic acid derivative thus obtained can form a salt, and the present invention also includes a case in which the product is obtained as a salt and a case in which the product is transformed to a salt after the reaction.

The fifth invention is a benzamidoacetic acid derivative or a salt thereof represented by the following formula, which is the starting material in the above-mentioned first invention and the starting material or the product in the above-mentioned fourth invention:

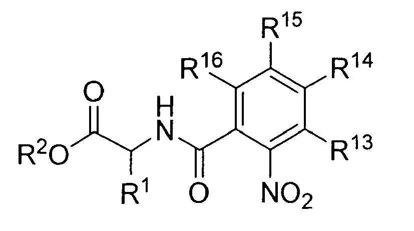

In the formula, R¹ represents a hydrogen atom or a C₁-C₆ alkyl group; R² represents a hydrogen atom or an ester-type protecting group; and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time.

Because this compound may contain a carboxyl group, it may form various kinds of salts, and the present invention also includes such salts.

When R¹ represents a C₁-C₆ alkyl group, there may exist enantiomers with respect to the carbon atom bonding to R¹. The present invention includes any of the R-form, the S-form and mixtures thereof.

The sixth invention is an acetamidopyrrolidine derivative or a salt thereof represented by the following formula, which is the product of the above-mentioned first invention and the starting material in the above-mentioned second invention.

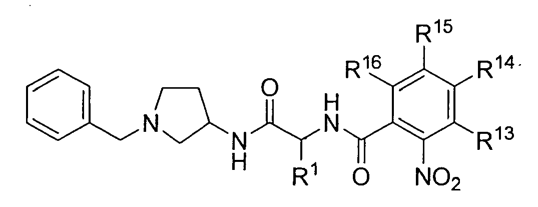

In the formula, R¹ represents a hydrogen atom or a C₁-C₆ alkyl group and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time.

Because this compound may contain a basic nitrogen, it may form various kinds of salts, and the present invention also includes such salts.

Further, because this compound has a chiral carbon in the pyrrolidine ring, there may exist enantiomers. The present invention includes any of the R-form, the S-form and mixtures thereof. Further, when R¹ represents a C₁-C₆ alkyl group, there may exist enantiomers with respect to the carbon atom bonding to R¹. The present invention includes any of the R-form, the S-form and mixtures thereof.

In the present description, the "ester-type protecting group" as R² or R²¹ means a group that can form an ester group together with the oxygen atom that bonds to R² or R²¹ and the carbonyl group adjacent thereto.

In the present description, the "C₁-C₆haloalkyl group" as R¹³, R¹⁴, R¹⁵ or R¹⁶ means a C₁-C₆ alkyl group substituted with a theoretically possible and arbitrary number of identical or different halogen atoms.

Similarly, the "C₁-C₆ haloalkoxy group" as R¹³, R¹⁴, R¹⁵ or R¹⁶ means a C₁-C₆ alkoxy group substituted with a theoretically possible and arbitrary number of identical or different halogen atoms.

### Best Mode for Carrying out the Invention

In any of the production methods and production intermediates relating to the present invention, R¹ represents a hydrogen atom or a C₁-C₆ alkyl group and R¹³ , R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time.

In particular, a hydrogen atom or a methyl group is preferred as R¹ and a hydrogen atom is particularly preferred. As for R¹³, R¹⁴, R¹⁵ and R¹⁶, it is preferred that any three of R¹³, R¹⁴, R¹⁵ and R¹⁶ are hydrogen atoms, and it is particularly preferred that R¹³, R¹⁴ and R¹⁶ are hydrogen atoms. In this case, furthermore, R¹⁵ is preferably a C₁-C₆ haloalkoxy group, particularly preferably a trifluoromethoxy group.

R² represents a hydrogen atom or an ester-type protecting group. As such an ester-type protection group there may be mentioned, for example, a methyl group, an ethyl group, a benzyl group and a tert-butyl group. Particularly, R² is preferably a hydrogen atom, a methyl group or an ethyl group.

The above-mentioned first invention is the first step in the following formula and a method for producing the above-mentioned sixth invention, the acetamidopyrrolidine derivative. It is preferred that this step is immediately followed by the reaction represented by the second step in the following formula, which is the above-mentioned second invention.

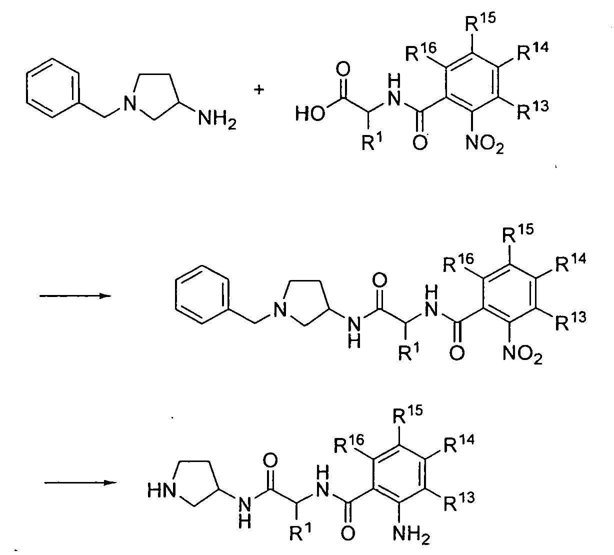

In the formula, R¹, R¹³, R¹⁴, R¹⁵ and R¹⁶ are as defined above.

In the above reaction formula, the first step is a condensation reaction. Because the starting materials, i.e., the aminopyrrolidine derivative and the benzamidoacetic acid derivative, contain an amino group and a carboxyl group, respectively, both of them can form a salt. Examples of salt of the aminopyrrolidine derivate include a salt with an inorganic acid, for example, hydrochloride. Examples of salt of the benzamidoacetic acid derivative include salt with an alkali metal or an alkaline-earth metal, for example, sodium salt. Those skilled in the art could readily find other specific examples.

Although the above formula represents reaction of a free base and a free acid as a representative case, the present invention also includes reactions in which a part of or all possible groups in one or both of these compounds are in a form of chemically possible salt. For example, the present invention also includes a case in which the staring material is a salt at the beginning of the reaction and converted to the corresponding free acid or free base in the reaction system to be subjected to the reaction.

The solvent used for this reaction is an aprotic solvent such as N,N-dimethylformamide, tetrahydrofuran and the like, or an ester-type solvent such as ethyl acetate. The condensing agent used here is, for example, 1-ethyl-3-(3-dimethylaminopropyl)carbodiimide or hydrochloride thereof, N,N'-dicyclohexylcarbodiimide, carbodimidazole, isobutyl chloroformate, diethylacetyl chloride or the like. If necessary, a basic additive such as triethylamine, N-methylmorpholine and the like may be used together. Particularly, it is more preferred that ethyl acetate is used as a solvent and 1-ethyl-3-(3-dimethylaminopropyl)carbodiimide is used as a condensing agent because of easiness of operation.

Furthermore, an additive may be added if necessary. As such an additive, for example, 1-hydroxy-1,2,3-benzotriazole or hydroxysuccinimide is used. The amount of the additive used is usually 0.1-1.0 equivalent. In particular, it is more preferred to use 0.1-0.2 equivalent of 1-hydroxy-1,2,3-benzotriazole.

The second step in the above reaction formula involves deprotection and reduction. That is, in this reaction, the benzyl group is reductively eliminated and at the same time the nitro group is reduced. As the reaction solvent, there may be mentioned, for example, alcoholic solvents such as methanol, ethanol, 2-propanol, and the like. A mixed solvent, for example, toluene-ethanol, may be also used. The reaction catalyst includes, for example, palladium catalysts such as 1-20% palladium on carbon and 1-20% palladium hydroxide (II) on carbon, platinum catalysts such as 1-10% platinum on carbon and the like. Particularly, 3-5% palladium on carbon is preferred. As such palladium catalysts or platinum catalysts, although either dried form or water-containing form may be used, it is preferred to use water-containing form in terms of the safety in industrial production processes. As a hydrogen source, there may be mentioned, for example, hydrogen gas, formic acid, ammonium formate and the like, among which hydrogen gas is preferred.

Here, in order to improve the reaction efficiency, various kinds of acid may be added. The acid used here includes, for example, hydrochloric acid, acetic acid, sulfuric acid, nitric acid, benzoic acid, boric acid, phosphoric acid, p-toluenesulfonic acid, methanesulfonic acid, citric acid, formic acid, tartaric acid, fumaric acid and malonic acid. In particular, use of acetic acid and nitric acid is more preferred. When the reaction is performed by adding such an acid, the product may be obtained as the corresponding salt depending on purification procedures.

Thus, the benzyl group is reductively eliminated and the nitro group is reduced at the same time. This is a characteristic point of the second invention. In addition, the sixth invention, acetamidopyrrolidine derivative, is characterized in that it can serve as a starting material for this type of reaction. Furthermore, achievement of synthesis of such a compound is a characteristic feature of the first invention.

The third invention is a production method of a benzamidoacetic acid derivative represented by the following formula.

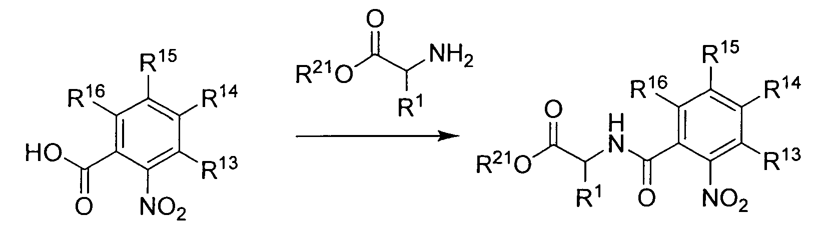

Here, the definitions and preferred examples of R¹, R¹³, R¹⁴, R¹⁵ and R¹⁶ are as described above. R²¹ represents an ester-type protecting group, and as specific examples thereof, the same groups as mentioned for the above-described ester-type protecting group R² may be mentioned as preferred groups. Among them, a methyl group and an ethyl group are preferred.

This step is a condensation reaction of a nitrobenzoic acid and an amino acid ester derivative.

Because the starting materials, i.e., the nitrobenzoic acid derivative and the amino acid ester derivative, contain a carboxyl group and an amino group, respectively, both of them can form a salt. Examples of salt of the nitrobenzoic acid derivative include salts with an alkaline metal or an alkaline earth metal, for example, sodium salt. Examples of salt of the amino acid ester derivative include salts with an inorganic acid, for example, hydrochloride. Those skilled in art could mention other specific examples.

Although the above reaction formula shows reaction of a free acid and a free base as a representative case, the present invention also includes reactions in which a part or all of the possible groups in one or both of the starting materials are in a form of chemically possible salt. For example, the present invention also includes a case in which the staring material is a salt at the beginning of the reaction and converted to the corresponding free acid or free base in the reaction system to be subjected to the reaction.

The solvent used for this reaction is an aprotic solvent, such as tetrahydrofuran, tert-butyl ethyl ether and the like, an ester-type solvent such as ethyl acetate or the like. A mixed solvent, such as a mixture of tert-butyl ethyl ether and methanol, may be used. As the condensing agent, there may be used, for example, 1-ethyl-3-(3-dimethylaminopropyl)carbodiimide or hydrochloride thereof, N,N'-dicyclohexylcarbodiimide, carbodimidazole, isobutyl chloroformate and the like. If necessary, a basic additive such as triethylamine and potassium carbonate may be used together. It is particularly preferred that ethyl acetate is used as a solvent, N,N'-dicyclohexylcarbodiimide is used as a condensing agent, and potassium carbonate is used as a basic additive in terms of convenience in operation.

Furthermore, an additive may be added in the present production method, if necessary. As such an additive, there may be used, for example, 1-hydroxy-1,2,3-benzotriazole and hydroxysuccinimide. The amount added is usually 0.1-1.0 equivalent. Particularly, it is more preferred to use 0.5-1.0 equivalent of 1-hydroxy-1,2,3-benzotriazole.

The fourth invention is a production method represented by the following formula.

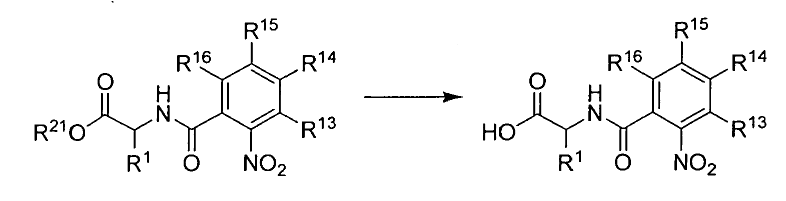

Here, the definitions and preferred examples of R¹, R¹³, R¹⁴, R¹⁵, R¹⁶ and R²¹ are as described above.

This step is a deprotection reaction of the ester-type protecting group. When the ester-type protecting group used is a methyl group, an ethyl group, a benzyl group or the like, for example, the ester can be hydrolyzed with an alkali in methanol, tetrahydrofuran, tert-butyl ethyl ether, water or a mixture thereof. As such an alkaline, there may be mentioned, for example, 0.1-2 M aqueous solution of sodium hydroxide. When a tert-butyl group is used as the protecting group, for example, the ester can also be hydrolyzed with an acid.

An example of preferred reactions in combination of the first invention to the fourth invention described above can be summarized as the following formula, that is, the reactions from the first step to the fourth step in the following formula. When the product is further subjected to the reaction represented by the fifth step in the following formula, one (compound A) of the above-described compounds described in WO 99/25686 pamphlet can be obtained.

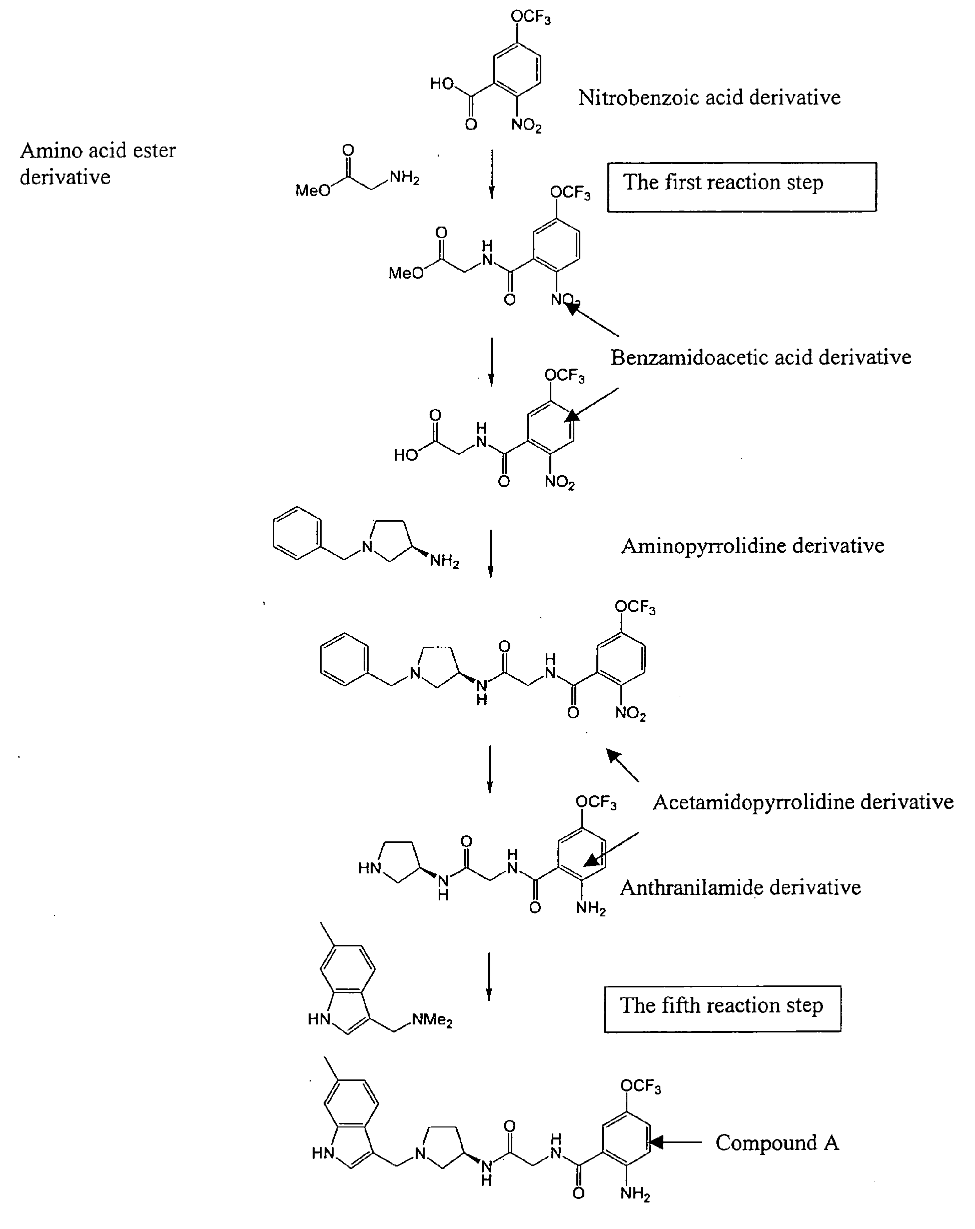

Compound A, which is obtained by the reaction represented by the fifth step in the above formula, is a particularly valuable compound as a compound having good balance between chemokine receptor antagonist activity and properties desired as a pharmaceutical agent such as in vivo pharmacokinetics, safety, chemical properties and the like, among the above-described compounds described in the pamphlet of WO 99/25686.

Furthermore, the present invention also includes a part or whole of the steps in the above formula, for example, the following methods for synthesizing compound A.

### [Method 1 for synthesizing compound A]

Method for synthesizing compound A, wherein the reaction of the fourth step in the above formula is performed and subsequently the reaction of the fifth step in the above formula is performed.

### [Method 2 for synthesizing compound A]

Method for synthesizing compound A, wherein the reaction of the third step in the above formula, the reaction of the fourth step in the above formula and the reaction of the fifth step in the above formula are sequentially performed.

### [Method 3 for synthesizing compound A]

Method for synthesizing compound A, wherein the reaction of the second step in the above formula, the reaction of the third step in the above formula, the reaction of the fourth step in the above formula and the reaction of the fifth step in the above formula are sequentially performed.

### [Method 4 for synthesizing compound A]

Method for synthesizing compound A, wherein the reaction of the first step in the above formula, the reaction of the second step in the above formula, the reaction of the third step in the above formula, the reaction of the fourth step in the above formula and the reaction of the fifth step in the above formula are sequentially performed.

Here, it is needless to say that processing steps such as a purification step, a concentration step and a solvent exchange step may be added between reactions of any steps in the above formula in these methods for synthesizing compound A.

The benzamidoacetic acid derivative relating to the above-mentioned fifth invention may contain a carboxyl group and may form a salt if chemically possible. For example, there may be mentioned sodium salt, potassium salt and the like.

This compound shows good crystallinity and advantageously can readily be prepared with high purity. Further, it is excellent in storability because of stability at room temperature. This feature also provides an advantage that purification in the subsequent reaction can be omitted, which is suitable for synthesis in an industrial scale.

The acetamidopyrrolidine derivative relating to the sixth invention also shows good crystalline and advantageously can readily be prepared with high purity. It is excellent in storability because of stability at room temperature. Further, it is also advantageous that the nitro group in this compound can be concurrently reduced during the subsequent step, which is a step of reductively eliminating the benzyl group (the second invention). Namely, the two reactions can be performed in one pot from the highly pure acetamidopyrrolidine derivative, which is favored in terms of production costs. As another advantage, the anthranilamide derivative formed is also obtained in high purity, and therefore it can be efficiently used for the subsequent reaction without purification.

As described above, the benzamidoacetic acid derivative relating to the fifth invention and the acetamidopyrrolidine derivative relating to the sixth invention show good crystallinity and are obtained with high purity, thereby making it possible to eliminate purification procedures in each of the subsequent reaction steps, which is suitable for synthesis in an industrial scale. Further, one may properly judge after which step purification should be performed, considering efficiency of purification, yields and the like, and thus flexibly adapt the procedures in industrial processing.

The characteristics of the above-described present invention, i.e., the first invention to the sixth invention, markedly appear particularly in synthesizing compound A.

That is, the fourth reaction step in the above formula, which is common to the above methods 1 to 4 for synthesizing compound A, has an advantage that the benzyl group is reductively eliminated and the nitro group is simultaneously reduced, which is advantageous in terms of production costs. In addition, it is also advantageous that the fifth reaction step in the above formula can efficiently be performed without purification after this one-pot synthesis. This point is characteristic of the second invention.

Besides this, the third reaction step in the above formula, which is common to the above methods 2 to 4 for synthesizing compound A, yields the acetamidopyrrolidine derivative relating to the sixth invention. This compound shows good crystallinity and advantageously can readily be prepared with high purity. Further, it is stable at room temperature and hence excellent in storability. This point is characteristic of the first invention.

Furthermore, the second reaction step in the above formula, which is common to the above methods 3 and 4 for synthesizing compound A, yields the benzamidoacetic acid derivative (wherein R² is a hydrogen atom) relating to the fifth invention. This compound also shows good crystallinity and advantageously can readily be prepared with high purity. It is also stable at room temperature and hence excellent in storability. Such properties enable to omit purification in the third reaction step in the above formula. This point is characteristic of the fourth invention.

Similarly, the first reaction step in the above formula, which is used in the above method 4 for synthesizing compound A, also yields the benzamidoacetic acid derivative (wherein R² is an ester-type protecting group) relating to the fifth invention. This compound also shows good crystallinity and has an advantage that material with high purity can readily be prepared. It is also stable at room temperature and hence excellent in storability. Such properties enable to omit purification in the second reaction step in the above formula, which is characteristic of the third invention.

From the above, for example, the reactions from the first steps to the third step in the above formula can sequentially be performed without purification procedures. Further, the reactions from the fourth step to the fifth step in the above formula can also be performed without purification procedures.

In the following, specific examples of the present invention are described as Examples. The other compounds of the present invention can also be synthesized with reference to these examples. From viewpoints of product yields, production costs, purity and the like, it is preferred to optimize reaction conditions such as reaction agent, reaction solvent, reaction temperature, reaction time, substrate concentration, and the like beforehand. The optimization can be easily performed by those skilled in the art on the basis of the present description and technical common knowledge, although it is not indispensable in carrying out the present invention.

### Examples

The present invention will be described in more detail with Examples below. However, the present invention is not limited to these examples.

### [Example 1]

### Synthesis of methyl(2-nitro-5-trifluoromethoxybenzamido)acetate

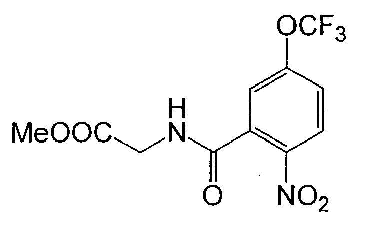

2-Nitro-5-trifluoromethoxybenzoic acid (10.53 g) was dissolved in ethyl acetate (200 mL). Here were added glycine methyl ester hydrochloride (5.79 g), N,N'-dicyclohexylcarbodiimide (9.52 g) and 1-hydroxy-1,2,3-benzotriazole hydrate (5.67 g). After that, potassium carbonate (11.59 g) was added and the reaction was carried out at 25°C for 2 h. After the reaction, water (50 mL) was added to stop the reaction, insoluble material was filtered off and water (50 mL) was added to the filtrate for separation. The organic layer was washed with a saturated aqueous solution (200 mL) of sodium hydrogen carbonate and the organic solvent was evaporated to obtain the title compound (12.90 g).
¹H NMR (200 MHz, DMSO-d₆, TMS standard): δ 3.35 (s, 3H), 3.69 (s, 2H), 4.06 (d, J = 5.8 Hz, 2H), 7.57 (brs, 1H), 7.73 (dd, J = 2.6 Hz and 1.2 Hz, 1H), 8.23 (d, J = 9.0 Hz, 1H), 9.3 (t, J = 5 .8 Hz, 1H)

### [Example 2]

### Synthesis of (2-nitro-5-trifluoromethoxybenzamido)acetic acid

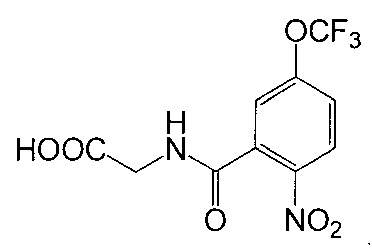

Methyl (2-nitro-5-trifluoromethoxybenzamido)acetate (6.18 g) was dissolved in tert-butyl methyl ether (200 mL) and to this solution, a 1 M aqueous solution (39.8 mL) of sodium hydroxide was added. After the reaction was performed at room temperature for 2 h, water (100 mL) was added for separation. To the aqueous layer separated, 2 M hydrochloric acid (25 mL) was added to precipitate crystals, which were collected by filtration to obtain the title compound (5.52 g).
¹H NMR (400 MHz, CDCl₃-d₆, TMS standard): δ 3.96 (d, J = 5.8 Hz, 2H), 7.56 (dd, J = 2.6 Hz and 0.8 Hz, 1H), 7.71-7.78 (m, 1H), 8.22 (d, J = 9.0 Hz, 1H), 9.22 (t, J = 5.8 Hz, 1H)

### [Example 3]

### Synthesis of ethyl (2-nitro-5-trifluoromethoxybenzamido)acetate

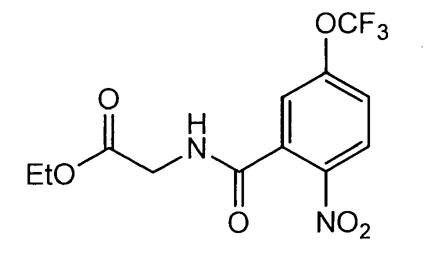

2-Nitro-5-trifluoromethoxybenzoic acid (6.47 g) was dissolved in ethyl acetate (130 mL). Here were added glycine ethyl ester hydrochloride (3.96 g), N,N'-dicyclohexylcarbodiimide (5.85 g) and 1-hydroxy-1,2,3-benzotriazole hydrate (3.94 g). After that, potassium carbonate (7.12 g) was added and the reaction was carried out at 25°C for 2 h. After the reaction, water (60 mL) was added to stop the reaction, insoluble material was filtered off, and water (50 mL) was added to the filtrate for separation. The organic layer was washed with a saturated aqueous solution (100 mL) of sodium hydrogen carbonate and then the organic solvent was evaporated to obtain the title compound (8.66 g).
¹H NMR (200 MHz, DMSO-d₆, TMS standard): δ 1.33 (t, J = 7.2 Hz, 3H), δ 4.25 (d, J = 5.2 Hz, 2H), δ 4.27 (q, J = 7.2 Hz, 2H), 7.27 (br, 1H), 7.39-7.43 (m, 3H), 8.18 (d, J = 5.2 Hz, 1H)

### [Example 4]

### Synthesis of (2-nitro-5-trifluoromethoxybenzamido)acetic acid

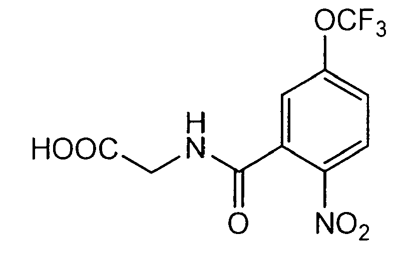

Ethyl (2-nitro-5-trifluoromethoxybenzamido)acetate (1.00 g) was dissolved in tert-butyl methyl ether (15 mL) and to this solution, a 1M aqueous solution (5.95 mL) of sodium hydroxide was added. After the reaction was performed at room temperature for 2 h, water (10 mL) was added for separation. To the aqueous layer separated, 2 M hydrochloric acid (3.72 mL) was added to precipitate crystals, which were collected by filtration to obtain the title compound (0.87 g).
¹H NMR (200 MHz, DMSO-d₆, TMS standard): δ 3.96 (d, J = 5.8 Hz, 2H), 7.56 (dd, J = 2.6 Hz and 0.8 Hz, 1H), 7.71-7.78 (m, 1H), 8.22 (d, J = 9.0 Hz, 1H), 9.22 (t, J = 5.8 Hz, 1H)

### [Example 5]

### Synthesis of methyl (2-nitro-5-trifluoromethoxybenzamido)acetate

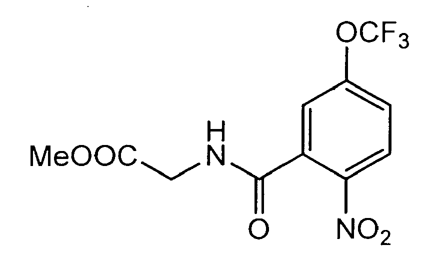

2-Nitro-5-trifluoromethoxybenzoic acid (20.00 g) was dissolved in tetrahydrofuran (400 mL). Here were added 1-hydroxy-1,2,3-benzotriazole hydrate (12.20 g) and N,N'-dicyclohexylcarbodiimide (18.08 g). After that, glycine methyl ester hydrochloride (11.00 g) and potassium carbonate (22.01 g) were added and the reaction was carried out at 25°C for 3 h. After the reaction, insoluble material was filtered off to obtain a tetrahydrofuran solution containing the title compound.

### [Example 6]

### Synthesis of (2-nitro-5-trifluromethoxybenzamido)acetic acid

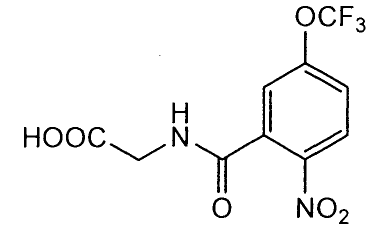

To the tetrahydrofuran solution (400 mL) containing methyl (2-nitro-5-trifluoromethoxybenzamido)acetate obtained in Example 5, a 0.5 M aqueous solution (191 mL) of sodium hydroxide was added. After the reaction was performed at room temperature for 2 h, the organic solvent was concentrated and tert-butyl methyl ether (200 mL) was added here for separation and the aqueous layer was collected. To this aqueous layer, 2 M hydrochloric acid (59.7 mL) was added to precipitate crystals, which were extracted by adding ethyl acetate (400 mL) to obtain an ethyl acetate solution containing the title compound.

### [Example 7]

### Synthesis of (R)-3-[2-(2-nitro-5-trifluoromethoxybenzamido)acetamido]-1-benzylpyrrolidine

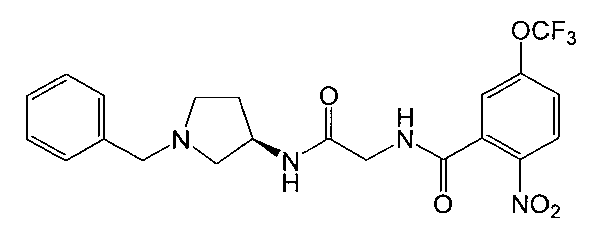

To the ethyl acetate solution (400 mL) containing 2-(2-nitro-5-trifluoromethoxybenzamido)acetic acid obtained in Example 6, were added (R)-1-benzyl-3-aminopyrrolidine (15.34 g), 1-hydroxy-1,2,3-benzotriazole (1.22 g), 1-ethyl-3-(3-dimethylaminopropyl)carbodiimide hydrochloride (16.68 g), and the resultant mixture was stirred at 40°C for 4 h. After the reaction, water (200 mL) was added to stop the reaction and water (200 mL) was added for separation. The organic layer was washed with a saturated aqueous solution (300 mL) of sodium hydrogen carbonate and dried over anhydrous sodium sulfate. After the drying agent was filtered off, the filtrate was concentrated under reduced pressure to obtain the title compound (33.39 g).
¹H NMR (200 MHz, DMSO-d₆, TMS standard): δ 1.43-1.59 (m, 1H), 1.91-2.12 (m, 1H), 2.22-2.62 (m, 4H), 3.48 (s, 2H), 3.76 (d, J = 5.8 Hz, 2H), 4.09-4.13 (m, 1H), 7.10-7.24 (m, 5H), 7.53-7.54 (m, 1H), 7.61-7.67 (m, 1H), 7.98 (d, J = 7.2 Hz, 1H), 8.13 (d, J = 8.8 Hz, 1H), 8.95 (t, J = 5.8 Hz, 1H)

### [Example 8]

### Synthesis of (R)-3-[2-(2-nitro-5-trifluoromethoxybenzamido)acetamido]-1-benzylpyrrolidine

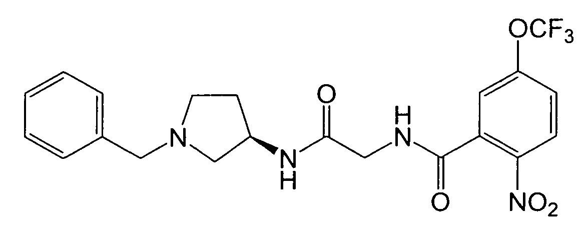

2-(2-Nitro-5-trifluoromethoxybenzamido)acetic acid (12.34 g) was dissolved in ethyl acetate (200 mL). To this solution, were added (R)-1-benzyl-3-aminopyrrolidine (7.76 g), 1-hydroxy-1,2,3-benzotriazole (0.54 g) and 1-ethyl-3-(3-dimethyaminopropyl)carbodiimide hydrochloride (8.44 g), and the resultant mixture was stirred at 40°C for 4 h. After the reaction, water (100 mL) was added to stop the reaction and water (50 mL) was added for separation. The organic layer was washed with a saturated aqueous solution (200 mL) of sodium hydrogen carbonate, further washed with a saturated aqueous solution (200 mL) of sodium chloride and dried over anhydrous sodium sulfate. After the drying agent was filtered off, the filtrate was concentrated under reduced pressure to obtain the title compound (17.56 g).
¹H NMR (200 MHz, DMSO-d₆, TMS standard): δ 1.43-1.59 (m, 1H), 1.91-2.12 (m, 1H), 2.22-2.62 (m, 4H), 3.48 (s, 2H), 3.76 (d, J = 5.8 Hz, 2H), 4.09-4.13 (m, 1H), 7.10-7.24 (m, 5H), 7.53-7.54 (m, 1H), 7.61-7.67 (m, 1H), 7.98 (d, J = 7.2 Hz, 1H), 8.13 (d, J = 8.8 Hz, 1H), 8.95 (t, J = 5.8Hz, 1H)

### [Example 9]

### Synthesis of (R)-3-[2-(2-amino-5-trifluoromethoxybenzamido)acetamido]pyrrolidine

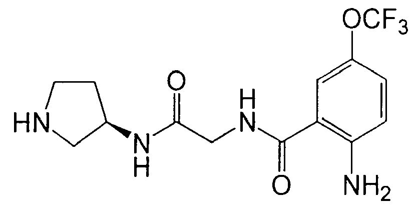

Ethanol (54 mL) was added to 10% palladium on carbon (0.50 g) and (R)-3-[2-(2-nitro-5-trifluoromethoxybenzamido)acetamido]-1-benzylpyrrolidine (5.00 g). After acetic acid (2.46 mL; 2 equivalents) was added here, the mixture was stirred at 30°C for 3 h while hydrogen gas was passed through the reaction system. The reaction solution obtained was filtered through a celite pad and the filtrate was concentrated under reduced pressure to obtain the title compound (4.35 g) as diacetate salt. After that, the free base was obtained by neutralization and extraction.
¹H NMR (200 MHz, DMSO-d₆, TMS standard): δ 1.50-1.63 (m, 1H), 1.84-2.01 (m, 1H), 1.86 (s, 3H), 2.60 (dd, J = 4.8 Hz, 11.4 Hz, 1H), 2.75-3.01 (m, 3H), 3.77 (d, J = 5.5 Hz, 2H), 4.08-4.21 (m, 1H), 6.65 (brs, 2H), 6.76 (d, J = 8.8 Hz, 1H), 7.17 (brd, J = 8.8 Hz, 1H), 7.55 (d, J = 2.2 Hz, 1H), 8.16 (d, J = 7.0 Hz, 1H), 8.57 (t, J = 5.5 Hz, 1H), 10.73 (brs, 1H)

### [Example 10]

### Synthesis of (R)-3-[2-(2-amino-5-trifluoromethoxybenzamido)acetamido]pyrrolidine

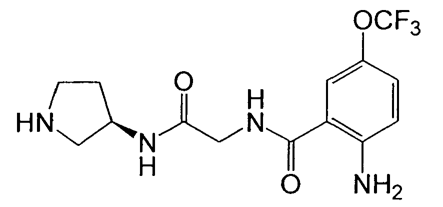

Ethanol (54 mL) was added to 10% palladium on carbon (0.50 g) and (R)-3-[2-(2-nitro-5-trifluoromethoxybenzamido)acetamido]-1-benzylpyrrolidine (5.00 g). After 60% nitric acid (1.63 mL; 2 equivalents) was added here, the mixture was stirred at temperatures from 30°C to 60°C for 3 h while hydrogen gas was passed through the reaction system. The reaction solution obtained was filtered through a celite pad and the filtrate was concentrated under reduced pressure to obtain the title compound (5.06 g) as dinitrate salt.

### [Example 11]

### Synthesis of (R)-3-[2-(2-amino-5-trifluoromethoxybenzamido)acetamido]pyrrolidine

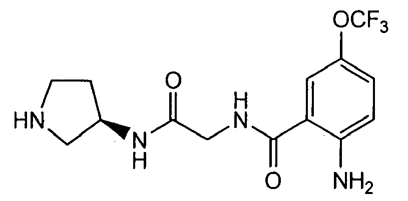

Ethanol (1070 mL) was added to 5% palladium on carbon (5 g) and (R)-3-[2-(2-nitro-5-trifluoromethoxybenzamido)acetamido]-1-benzylpyrrolidine (100 g). The resultant mixture was stirred at 20°C for 8 h while hydrogen gas was passed through the reaction system, and then 60% nitric acid (32.6 mL; 2 equivalents) was added and the mixture was stirred at 60°C for 2 h. The reaction solution obtained was filtered through a celite pad to obtain nitrate salt of the title compound as an ethanolic solution.

### [Example 12]

### Synthesis of (R)-3-[2-(2-amino-5-trifluoromethoxybenzamido)acetamido]-1-(6-methylindol-3-ylmethyl)pyrrolidine

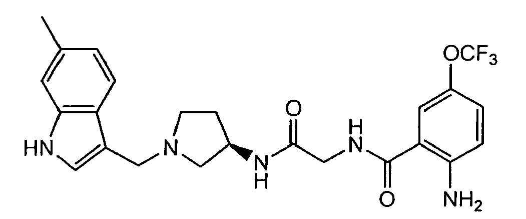

To the ethanol solution (1070 mL) containing (R)-3-[2-(2-amino-5-trifluoromethoxybenzamido)acetamido]pyrrolidine nitrate obtained in Example 11, a 2 mol/L aqueous solution of potassium carbonate (118 mL; 1.1 equivalents) was added and the mixture was stirred for 20 min. Next, the bath temperature was slowly increased up to 70°C under reduced pressure to remove 580 mL of ethanol. After that, toluene (2.1 L) and 6-methylgramine (44.4 g; 1.1 equivalents) were added and the mixture was heated with stirring under reduced pressure in an oil bath whose temperature was 100°C. When the temperature of the reaction solution reached 80°C, the bath temperature was increased to 120°C and toluene (500 mL) was added to the reaction solution, this solution was heated for 8 h and then the reaction was stopped. After the solution was gradually cooled down to room temperature, water (1 L) was added here, the mixture was stirred for 30 min and slurry-like crystals obtained were collected with a centrifugal separator. The crystals were washed with toluene (500 mL) and water (500 mL) and then dried at 40°C in high vacuum for 2 days to obtain the title compound (103.48 g) as white crystals.
¹H NMR (200 MHz, DMSO-d₆, TMS standard): δ 1.48-1.62 (m, 1H), 1.99-2.15 (m, 1H), 2.28-2.46 (m, 2H), 2.37 (s, 3H), 2.56-2.69 (m, 2H), 3.33 (s, 2H), 3.75 (d, J = 5.9 Hz, 2H), 4.06-4.22 (m, 1H), 6.64 (brs, 2H), 6.76 (d, J = 9.2 Hz, 1H), 6.79 (d, J = 8.1 Hz, 1H), 7.12-7.19 (m, 3H), 7.47 (d, J = 8.1 Hz, 1H), 7.52 (d, J = 2.9 Hz, 1H), 8.04 (d, J = 7.3 Hz, 1H), 8.51 (d, J = 5.9 Hz, 1H), 10.73 (brs, 1H)

### Industrial Applicability

The benzamidoacetic acid derivatives and the acetamidopyrrolidine derivatives of the present invention are used as intermediates for pharmaceuticals. Further, the production methods of the present invention provide intermediates for producing pharmaceuticals.

## Claims

1. A production method of an acetamidopyrrolidine derivative or a salt thereof comprising the reaction step represented by the following formula: 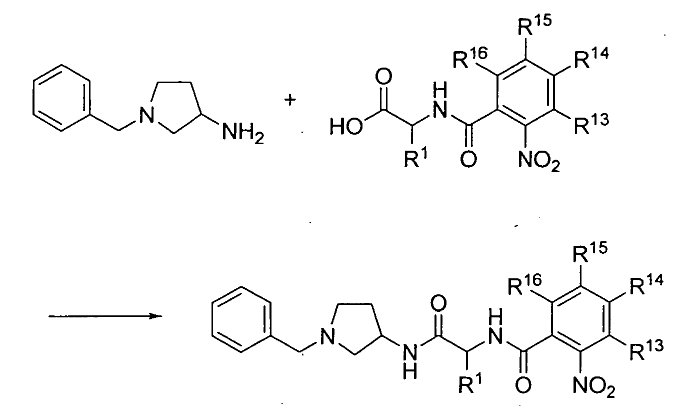 wherein R¹ represents a hydrogen atom or a C₁-C₆ alkyl group and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time; wherein one or both of the aminopyrrolidine derivative and the benzamidoacetic acid derivative used as the starting materials may be in a form of salt.

2. A production method of an acetamidopyrrolidine derivative or a salt thereof comprising the reaction step represented by the following formula: 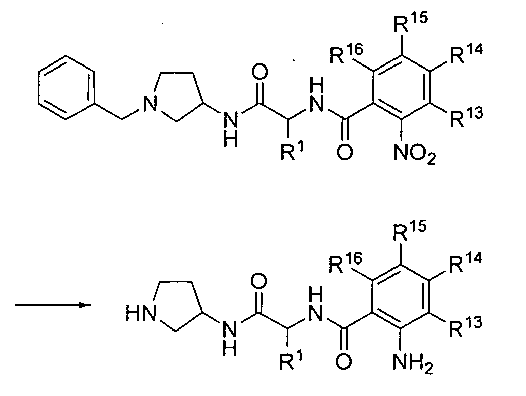 wherein R¹ represents a hydrogen atom or a C₁-C₆ alkyl group and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time; wherein the acetamidopyrrolidine derivative used as the starting material may be in a form of salt.

3. A production method of an acetamidopyrrolidine derivative or a salt thereof comprising the reaction steps represented by the following formula: 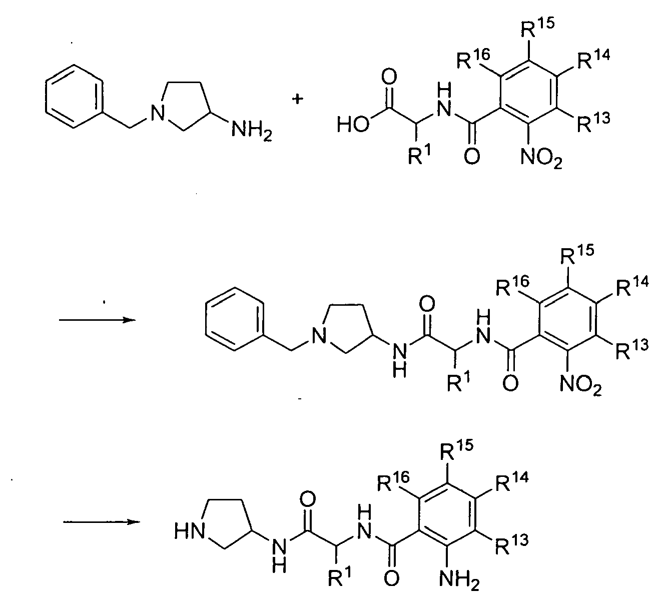 wherein R¹ represents a hydrogen atom or a C₁-C₆ alkyl group and R¹³, R¹⁴ , R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time; wherein one or both of the aminopyrrolidine derivative and the benzamidoacetic acid derivative used as the starting materials of the first reaction step may be in a form of salt.

4. The production method according to any of claims 1 to 3, wherein R¹ is a hydrogen atom.

5. The production method according to any of claims 1 to 4, wherein any three of R¹³, R¹⁴, R¹⁵ and R¹⁶ are hydrogen atoms.

6. The production method according to claim 5, wherein R¹³, R¹⁴ and R¹⁶ are hydrogen atoms.

7. The production method according to claim 6, wherein R¹⁵ is a C₁-C₆ haloalkoxy group.

8. The production method according to claim 6, wherein R¹⁵ is a trifluoromethoxy group.

9. A production method of a benzamidoacetic acid derivative comprising the reaction step represented by the following formula: 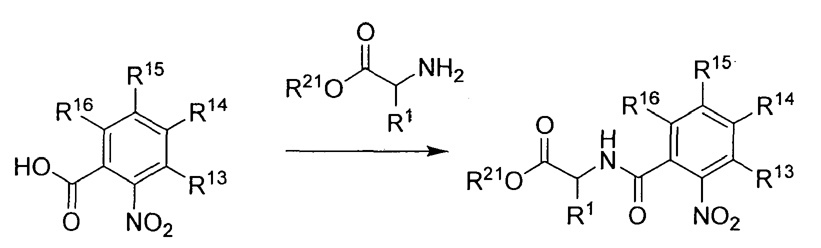 wherein R¹ represents a hydrogen atom or a C₁-C₆ alkyl group; R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group; and R²¹ represents an ester-type protecting group, excluding a case in which all of R¹³, R¹⁴, R¹⁵ and R¹⁶ are hydrogen atoms at the same time; wherein one or both of the nitrobenzoic acid derivative and the amino acid ester derivative used as the starting materials may be in a form of salt.

10. A production method of a benzamidoacetic acid derivative or a salt thereof comprising the reaction step represented by the following formula: 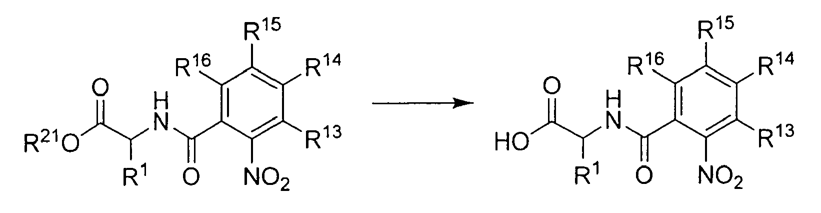 wherein R¹ represents a hydrogen atom or a C₁-C₆ alkyl group; R¹³ R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group; and R²¹ represents an ester-type protecting group, excluding a case in which all of R¹³, R¹⁴, R¹⁵ and R¹⁶ are hydrogen atoms at the same time.

11. A production method of a benzamidoacetic acid derivative or a salt thereof wherein a step comprising the reaction step according to claim 9 is performed and subsequently a step comprising the reaction step according to claim 10 is performed.

12. The production method according to any of claims 9 to 11, wherein R²¹ is a methyl group or an ethyl group.

13. The production method according to any of claims 9 to 12, wherein R¹ is a hydrogen atom.

14. The production method according to any of claims 9 to 13, wherein any three of R¹³, R¹⁴, R¹⁵ and R¹⁶ are hydrogen atoms.

15. The production method according to claim 14, wherein R¹³, R¹⁴ and R¹⁶ are hydrogen atoms.

16. The production method according to claim 15, wherein R¹⁵ is a C₁-C₆ haloalkoxy group.

17. The production method according to claim 15, wherein R¹⁵ is a trifluoromethoxy group.

18. A production method of a 2-nitrobenzoic acid derivative or a salt thereof comprising the reaction step represented by the following formula:

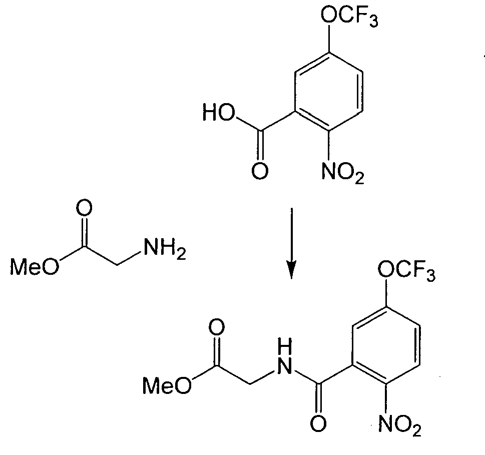

19. A production method of a benzamidoacetic acid derivative comprising the reaction steps represented by the following formula:

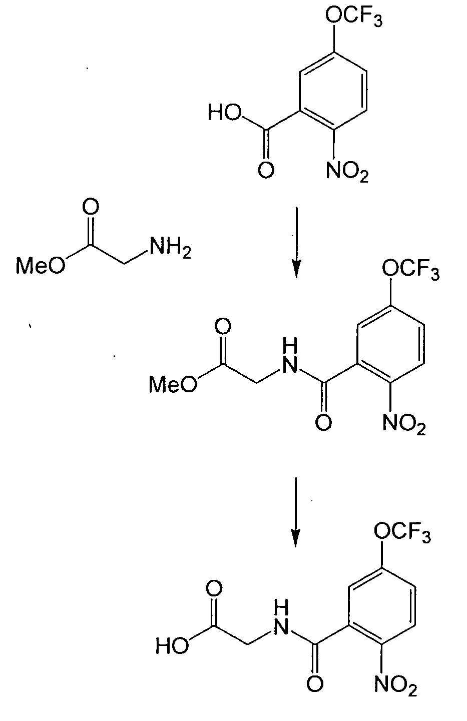

20. A production method of a benzamidoacetic acid derivative or a salt thereof comprising the reaction steps represented by the following formula:

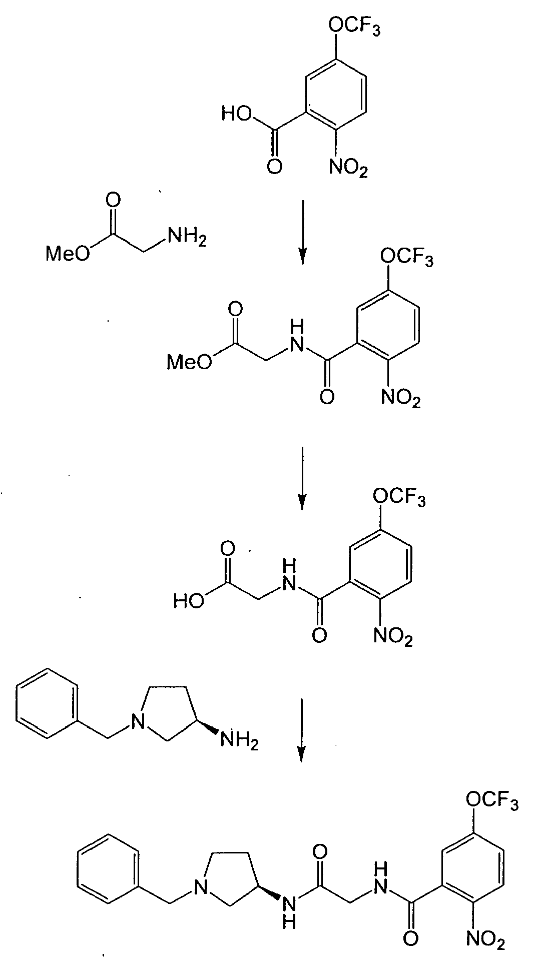

21. A production method of an acetamidopyrrolidine derivative or a salt thereof comprising the reaction steps represented by the following formula:

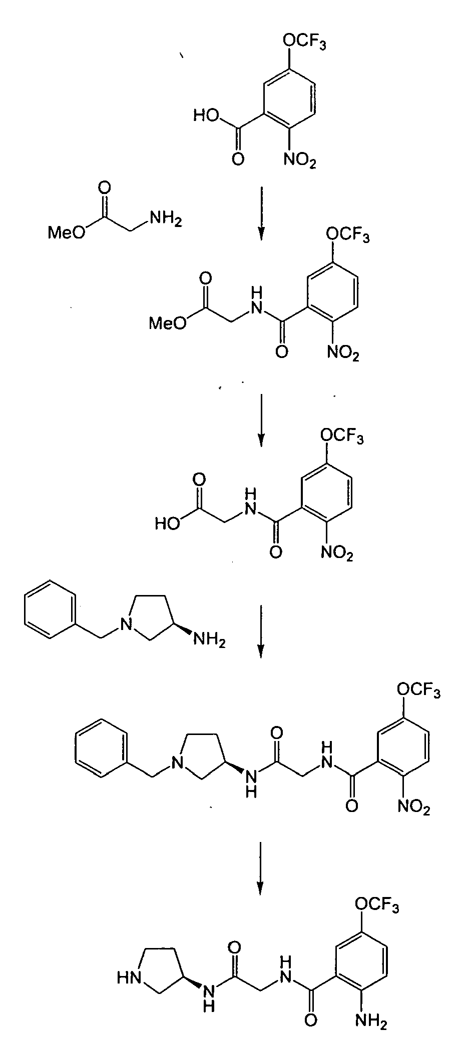

22. A production method of an acetamidopyrrolidine derivative or a salt thereof comprising the reaction steps represented by the following formula:

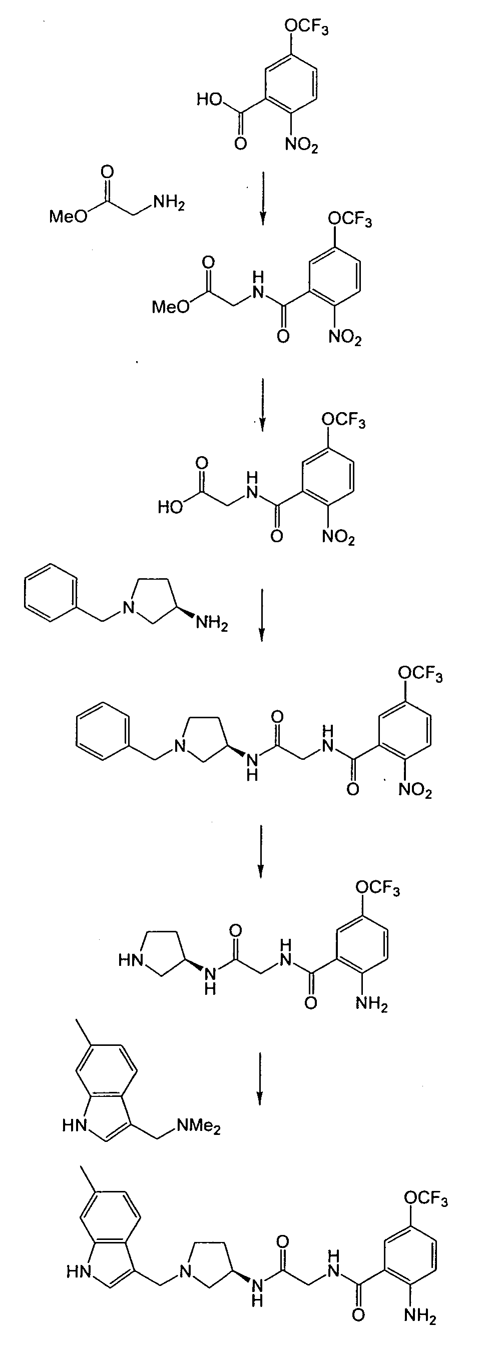

23. A compound represented by the following formula or a salt thereof: 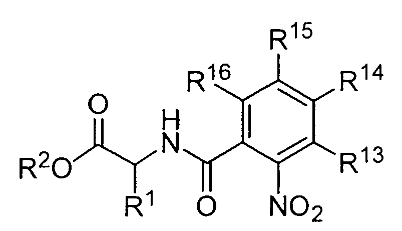 wherein R¹ represents a hydrogen atom or a C₁-C₆ alkyl group; R² represents a hydrogen atom or an ester-type protecting group; and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time.

24. The compound or a salt thereof according to claim 23, wherein R² is a methyl group, an ethyl group, a benzyl group or a tert-butyl group.

25. The compound or a salt thereof according to claim 23, wherein R² is a hydrogen atom.

26. The compound or a salt thereof according to claim 23, wherein R² is a methyl group or an ethyl group.

27. A compound represented by the following formula or a salt thereof: 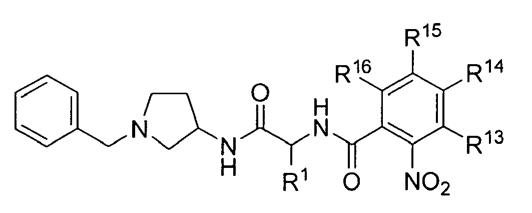 wherein R¹ represents a hydrogen atom or a C₁-C₆ alkyl group and R¹³, R¹⁴, R¹⁵ and R¹⁶ each represent independently a hydrogen atom, a halogen atom, a C₁-C₆ alkyl group, a C₁-C₆ alkoxy group, a hydroxyl group, a C₁-C₆ haloalkyl group or a C₁-C₆ haloalkoxy group, excluding a case in which all of R¹³, R¹⁴, R¹⁵ and R¹⁶ are hydrogen atoms at the same time.

28. The compound or a salt thereof according to any of claims 23 to 27, wherein R¹ is a hydrogen atom.

29. The compound or a salt thereof according to any of claims 23 to 28, wherein any three of R¹³, R¹⁴, R¹⁵ and R¹⁶ are hydrogen atoms.

30. The compound or a salt thereof according to claim 29, wherein R¹³, R¹⁴ and R¹⁶ are hydrogen atoms.

31. The compound or a salt thereof according to claim 30, wherein R¹⁵ is a C₁-C₆ haloalkoxy group.

32. The compound or a salt thereof according to claim 30, wherein R¹⁵ is a trifluoromethoxy group.
